# EUROPEAN PATENT APPLICATION

(11) **EP 3 754 666 A1**
(43) Date of publication of application: **23.12.2020**
(21) Application number: 19181472.2
(22) Date of filing: 20.06.2019
(51) Int. Cl.: G16H 30/40

(54) **MEDICAL IMAGE CHECK APPARATUS**

(71) Applicant: Koninklijke Philips N.V., 5656 AG Eindhoven (NL)
(72) Inventor: MAACK, Hanns-Ingo, 5656 AE Eindhoven (NL)
(74) Representative: de Haan, Poul Erik

(57) **Abstract**

The present invention relates to a medical image check apparatus (10). The apparatus comprises an input unit (20), a processing unit (30), and an output unit (40). The input unit is configured to provide a radiological image to the processing unit. The radiological image comprises image data associated with an anatomical feature of a patient. A descriptive label is associated with the radiological image. The processing unit is configured to identify the anatomical feature in the radiological image, the identification comprising utilization of an image processing algorithm. The processing unit is configured to determine a consistency between the identified anatomical feature in the radiological image and the descriptive label. The output unit is configured to output information on the basis of the determined consistency.

## Description

### FIELD OF THE INVENTION

The present invention relates to a medical image check apparatus, a method of checking a medical image, as well as to a computer program element and a computer readable medium.

### BACKGROUND OF THE INVENTION

Radiographers need to take different views of different anatomical features of patients, and keeping track of this information can be difficult and mistakes do happen, where an acquired image is incorrectly labelled.

EP2457512A1 describes a system and method for including and correcting subject orientation data in a digital radiographic image. A subject is positioned in an X-ray imaging system and an orientation of the subject is entered into the X-ray imaging system. The subject is imaged with an X-ray exposure to create an X-ray image of the subject. An operator reviews the X-ray image of the subject and subject orientation data. If the subject orientation data is not correct, the operator may correct the subject orientation data so that it matches the subject's anatomy during the X-ray exposure. The correct subject orientation data is then saved and is part of the X-ray image and Digital Imaging and Communications in Medicine (DICOM) header when sent to a Picture Archiving and Communication System (PACS).

US2010185459A1 describes systems and methods for X-ray image identification. The systems and methods associate patient information with image information. The method includes acquiring patient information from an identification member external to the digital X-ray detector and storing the patient information within the X-ray detector or on an image. The method further includes associating the patient information with the images acquired by the X-ray imaging system and communicating the acquired images with associated information to a host system when the x-ray detector is connected to the host system.

US2012002853A1 describes a system and method for image file header configuration. This involves retrieving one or more criterion for configuration of an image orientation parameter, configuring the image orientation parameter based on the one or more criterion, obtaining image data, and storing the image data in an image file. The image file has a header portion including the image orientation parameter. In an embodiment, the image orientation parameter may be modified from a default configuration. The one or more criterion may include user preference, modality restriction, system preference, and/or rule, for example. The method may further include saving the image file with the configured image orientation parameter. Additionally, the method may include displaying an image according to the configured image orientation parameter. The image may be automatically oriented for display based on the configured image orientation parameter.

However, it has been established that radiographers require further help.

### SUMMARY OF THE INVENTION

It would be advantageous to have improved means of providing information to a radiographer regarding the integrity of an acquired x-ray image. The object of the present invention is solved with the subject matter of the independent claims, wherein further embodiments are incorporated in the dependent claims. It should be noted that the following described aspects and examples of the invention apply also to the medical image check apparatus, the method of checking a medical image, as well as to the computer program element and a computer readable medium.

In a first aspect, there is provided a medical image check apparatus, comprising:
- an input unit;
- a processing unit; and
- an output unit.

The input unit is configured to provide a radiological image to the processing unit. The radiological image comprises image data associated with an anatomical feature of a patient. A descriptive label is associated with the radiological image. The processing unit is configured to identify the anatomical feature in the radiological image, the identification comprising utilization of an image processing algorithm. The processing unit is configured to determine a consistency between the identified anatomical feature in the radiological image and the descriptive label. The output unit is configured to output information on the basis of the determined consistency.

In this manner, a smart exam functionality is provided, where for example for a chest PA/AP image feedback is provided to the radiographer in case there are aspects that affect the specific image. Also, a determination can be made that a PA image has been correctly labelled and has not for example been labelled as a LAT image, and vice versa. This is very beneficial because these types of images are frequently acquired together, but the order in which they are acquired, which can lead to incorrect labelling. However, the apparatus can catch this mistake. Thus, a consistency check can be made as to whether the correct part of the anatomy has been imaged, whether it has been imaged in the correct orientation and/or view and/or state of rotation, whether the patient was in the correct "state" - such as having exhaled or inhaled.

In other words, and immediate and fool proof feedback mechanism is provided to aid the work of radiographers, through a check of the consistency between an X-ray image and the label.

To put this another way, the radiographer is helped because the medical image check apparatus - "anatomy checker" identifies the anatomy in the image and makes an alert if it does not match with describing labels.

Thus in this way, the correctness of projections is improved, it is assured that the right image was taken, it is assured that the labelling in a PACS is consistent (thereby aiding for example in ground truth label/image association), and post-processing is stabilized as anatomy-specific parameters will always match with the correct/real image.

In other words, the apparatus can determine that a part of the body was acquired with x-rays passing from the posterior of the body to the anterior and if the associated label is PA then it can be determined that the label is consistent. But on the other hand if the label was AP it can be determined that the label is not consistent. This can be achieved, for example because in AP view posterior aspect gives better shadow, while in PA view anterior aspect gives better more clearer shadow due to features that are closer to the detector having a clearer shadow.

In an example, the processing unit is configured to utilize the descriptive label to select at least one reference radiological image. The identification of the anatomical feature in the radiological image can then comprise a comparison between the radiological image and the at least one reference radiological image.

In other words, for example the apparatus can determine that a part of the body was acquired with x-rays passing from the posterior of the body to the anterior and if the associated label is PA then it can be determined that the label is consistent. Thus, part of the anatomy is acquired, for example a part of the anatomy acquired with in a PA view, whilst the label associated with the image is AP. Then, the database of images is used to provide one or more images of that part of the anatomy with an AP view, and the apparatus can immediate determine that the acquired image is not consistent, because it was acquired from the opposite direction.

In an example, if a determination is made that no reference radiological images are available in a local database, the processing unit is configured to query an external database to select the at least one reference radiological image.

In an example, the processing unit is configured to instruct that the local database is populated with the selected at least one reference radiological image from the external database.

Thus, a local projection database can be updated using images with projections that are not common (or known) to the apparatus. In this manner, the apparatus can be continually improved automatically in an efficient manner.

In an example, the radiological image comprises image data associated with the descriptive label.

In an example, the image data associated with the descriptive label comprises a digital overlay.

In an example, the image data associated with the descriptive label was acquired at the same time as image data associated with the anatomical feature of the patient by an image acquisition unit. The processing unit is configured to implement an image processing algorithm to identify the descriptive label.

In an example, identification of the descriptive label comprises utilization of a text recognition algorithm.

In an example, if the processing unit determines that the identified anatomical feature in the radiological image is consistent with the descriptive label, the processing unit is configured to control the output unit such that the output information comprises an indication that an anatomy and/or image check is ok.

In an example, the output information comprises details of at least one further image view of the anatomical feature to a view associated with the descriptive label.

In an example, if the processing unit determines that the identified anatomical feature in the radiological image is not consistent with the descriptive label, the processing unit is configured to control the output unit such that the output information comprises an indication that an anatomy and/or image check is not ok.

In an example, the output information comprises one or more of: a request to check the anatomical feature; a request to check a view direction of an image acquisition unit; an alternative descriptive label; a request to re-take a scan with matching positioning; a display of tutorial information.

In an example, the input unit is configured to receive an input from a user that the identified anatomical feature in the radiological image is consistent with the descriptive label.

In a second aspect, there is provided a method of checking a medical image, comprising:
- providing from an input unit to a processing unit a radiological image, wherein the radiological image comprises image data associated with an anatomical feature of a patient, and wherein a descriptive label is associated with the radiological image;
- identifying by the processing unit the anatomical feature in the radiological image, the identifying comprising utilizing an image processing algorithm;
- determining by the processing unit a consistency between the identified anatomical feature in the radiological image and the descriptive label; and
- outputting by an output unit output information on the basis of the determined consistency.

According to another aspect, there is provided a computer program element controlling one or more of the apparatuses as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a medical image check apparatus;
Fig. 2 shows a method of checking a medical image; and
Fig. 3 shows a representation of an X-ray image with descriptive labelling.

### DETAILED DESCRIPTION OF EMBODIMENTS

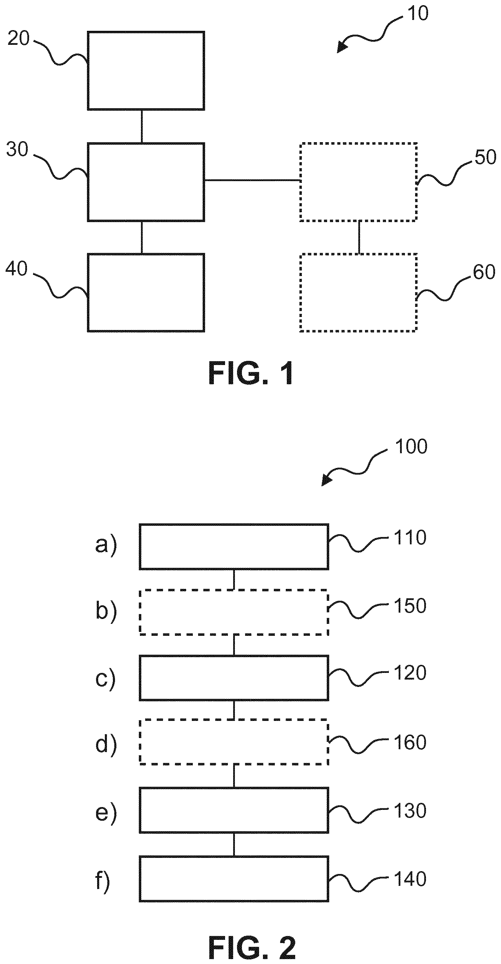

Fig. 1 shows an example of a medical image check apparatus 10, where essential features are represented in solid lines and optional features are represented in hashed lines. The apparatus comprises an input unit 20, a processing unit 30, and an output unit 40. The input unit is configured to provide a radiological image to the processing unit. The radiological image comprises image data associated with an anatomical feature of a patient. A descriptive label is associated with the radiological image. The processing unit is configured to identify the anatomical feature in the radiological image. The identification comprises utilization of an image processing algorithm. The processing unit is configured to determine a consistency between the identified anatomical feature in the radiological image and the descriptive label. The output unit is configured to output information on the basis of the determined consistency.

In an example, the determination of consistency comprises a determination that a RIS (Radiology Information System) code required in acquiring the radiological image does not fit with the identified anatomical feature.

In an example, identification of the anatomical feature includes an identification of the projection used in acquiring the image data.

In an example, the determination of consistency comprises a determination that a DICOM (Digital Imaging and Communications in Medicine) code describing the radiological image does not fit with the identified anatomical feature.

According to an example, the processing unit is configured to utilize the descriptive label to select at least one reference radiological image. The identification of the anatomical feature in the radiological image can then comprise a comparison between the radiological image and the at least one reference radiological image.

In an example, reference radiological images are held in a local database 50 also called a projection-database.

In an example, a local database in the form of a projection database is populated with using ground truth, where imagery is correctly associated with descriptive labels. This can be achieved manually, or through the use of artificial intelligence, for example utilizing machine learning algorithms.

According to an example, if a determination is made that no reference radiological images are available in a local database 50, the processing unit is configured to query an external database 60 to select the at least one reference radiological image.

According to an example, the processing unit is configured to instruct that the local database is populated with the selected at least one reference radiological image from the external database.

According to an example,n the radiological image comprises image data associated with the descriptive label.

According to an example, the image data associated with the descriptive label comprises a digital overlay.

In an example, the digital overlay is provided from DICOM projection information.

According to an example, the image data associated with the descriptive label was acquired at the same time as image data associated with the anatomical feature of the patient by an image acquisition unit. The processing unit is configured to implement an image processing algorithm to identify the descriptive label.

According to an example, identification of the descriptive label comprises utilization of a text recognition algorithm.

According to an example, if the processing unit determines that the identified anatomical feature in the radiological image is consistent with the descriptive label, the processing unit is configured to control the output unit such that the output information comprises an indication that an anatomy and/or image check is ok.

According to an example, the output information comprises details of at least one further image view of the anatomical feature to a view associated with the descriptive label.

According to an example, if the processing unit determines that the identified anatomical feature in the radiological image is not consistent with the descriptive label, the processing unit is configured to control the output unit such that the output information comprises an indication that an anatomy and/or image check is not ok.

According to an example, the output information comprises one or more of: a request to check the anatomical feature; a request to check a view direction of an image acquisition unit; an alternative descriptive label; a request to re-take a scan with matching positioning; a display of tutorial information.

According to an example, the input unit is configured to receive an input from a user that the identified anatomical feature in the radiological image is consistent with the descriptive label.

In an example, the processing unit is configured to update a local database on the basis of the user provided correction.

Fig. 2 shows a method 100 of checking a medical image in its basic steps, where essential steps are represented in solid lines and optional steps are represented in hashed lines. The method comprises:
- in a providing step 110, also referred to as step a), providing from an input unit to a processing unit a radiological image, wherein the radiological image comprises image data associated with an anatomical feature of a patient, and wherein a descriptive label is associated with the radiological image;
- in an identifying step 120, also referred to as step c), identifying by the processing unit the anatomical feature in the radiological image, the identifying comprising utilizing an image processing algorithm;
- in a determining step 130, also referred to as step e), determining by the processing unit a consistency between the identified anatomical feature in the radiological image and the descriptive label; and
- in an outputting step 140, also referred to as step f), outputting by an output unit output information on the basis of the determined consistency.

In an example, the determining of consistency comprises a determination that a RIS code required in acquiring the radiological image does not fit with the identified anatomical feature.

In an example, the determining of consistency comprises a determination that a DICOM code describing the radiological image does not fit with the identified anatomical feature.

In an example, the method comprises step b) selecting 150 by the processing unit at least one reference radiological image, the selecting comprising utilizing the descriptive label, and wherein step c) comprises comparing the radiological image and the at least one reference radiological image.

In an example, reference radiological images are held in a local database.

In an example, wherein in step b) if the processing unit determines determination that no reference radiological images are available in a local database, the processing unit queries an external database to select the at least one reference radiological image.

In an example, the method comprises populating the local database with the selected at least one reference radiological image from the external database.

In an example, the radiological image comprises image data associated with the descriptive label.

In an example, the image data associated with the descriptive label comprises a digital overlay.

In an example, the digital overlay is provided from DICOM projection information.

In an example, the image data associated with the descriptive label was acquired at the same time as image data associated with the anatomical feature of the patient by an image acquisition unit; and wherein the method comprises step d) identifying 160)by the processing unit the descriptive label comprising implementing an image processing algorithm.

In an example, identifying the descriptive label comprises utilization of a text recognition algorithm.

In an example, if in step e) it is determined that the identified anatomical feature in the radiological image is consistent with the descriptive label, step f) comprises controlling by the processing unit the output unit such that the output information comprises an indication that an anatomy and/or image check is ok.

In an example, the output information comprises details of at least one further image view of the anatomical feature to a view associated with the descriptive label.

In an example, if in step e) it is determined that the identified anatomical feature in the radiological image is not consistent with the descriptive label, step f) comprises controlling by the processing unit the output unit such that the output information comprises an indication that an anatomy and/or image check is not ok.

In an example, the output information comprises one or more of: a request to check the anatomical feature; a request to check a view direction of an image acquisition unit; an alternative descriptive label; a request to re-take a scan with matching positioning; a display of tutorial information.

In an example, method comprises receiving by the input unit an input from a user that the identified anatomical feature in the radiological image is consistent with the descriptive label.

In an example, the processing unit is configured to update a local database on the basis of the user provided correction.

Thus, in a percentage of examinations, it has been established that the real image does not fit to the RIS code that required making it or the DICOM code describing it. Often the radiographer simply confused something leading to this error. The medical image check apparatus and method of checking a medical image described here address this by determining a consistency between the acquired image and the descriptive label, and if there is no match this can be indicated to the radiographer.

The following is a list of 119 anatomical programs used in one hospital, and serves to demonstrate the possibilities for an acquired image to be miss-labelled.

| | |
|---|---|
| Abdomen pa decub | Coccyx ap |
| Abdomen ap | Crosswise Abdomen |
| Abdomen ap decub | Elbow ap |
| Abdomen children(L) ap | Elbow lat |
| Abdomen lat | Elbow oblique |
| Abdomen pa | Femur / Knee ap |
| Ankle ap | Femur / Knee lat |
| Ankle lat | Finger ap |
| Ankle oblique | Finger lat |
| Bilateral Knees AP | Finger oblique |
| Calcaneus axial | Foot ap |
| Calcaneus lat | Foot lat |
| Cervical spine ap | Foot oblique |
| Cervical spine extension | Forearm / Elbow ap |
| Cervical spine flexion | Forearm / Elbow lat |
| Cervical spine lat | Hand lat |
| Cervical spine lat crosswise | Hand oblique |
| Cervical spine oblique | Hand pa |
| Cervical spine swimmers | Hip / Femur ap |
| Cervical spine swimmers crosswise | Hip / Femur lat |
| Chest ap | Hip ap |
| Chest Decub AP | Hip med-lat |
| Chest Decub PA | Humerus / Shoulder ap |
| Chest lat | Humerus / Shoulder lat |
| Chest oblique | Knee ap |
| Chest pa | Knee axial |
| Chest PA Crosswise | Knee lat |
| Clavicle ap | Knee Tunnel |
| Clavicle axial | Lower leg / Ankle ap |
| | Lower leg / Ankle lat |
| Lower leg / Knee ap | Scapula lat |
| Lower leg / Knee lat | Shoulder ap |
| Lower leg children(M) ap | Shoulder axial |
| Lower leg children(S) ap | Shoulder oblique |
| Lower leg children(S) lat | Shoulder semiaxial |
| Lumbar spine ap | Shoulder trans-thoracic |
| Lumbar spine extension | SI JOINT LPO |
| Lumbar spine flexion | SI JOINT RPO |
| Lumbar spine L5 S1 | Sinuses / Waters |
| Lumbar spine lat | Sinuses Caldwell |
| Lumbar spine oblique | Sinuses Lat |
| Mandible Obilque RT | Skull / Waters |
| Mandible Oblique LT | Skull ap |
| Mandible PA | Skull lat |
| Mandible Towne | Skull pa |
| Nasal bone | Skull semiaxial / Towne |
| Nevicular View | Sonstige |
| Odontoid open mouth | Sternum lat |
| Odontoid Waters | Sternum pa oblique |
| Patella axial | Thoracic spine ap |
| Pelvis ap | Thoracic spine lat |
| Ribs 1-7 ap | Toes ap |
| Ribs 1-7 oblique | Toes lat |
| Ribs 1-7 pa | Toes oblique |
| Ribs 8-12 ap | Top Ten |
| Ribs 8-12 oblique | Untersuchung |
| Ribs 8-12 pa | Wrist lat |
| Sacrum ap | Wrist oblique |
| Sacrum lat | Wrist pa |
| Scapula ap | Zygomatic arch single |

Thus, during the X-ray examination of a patient, some errors may occur.

For example, the laterality may be miss-labelled, where the organ is annotated as "Right" but it is in fact "left"

A chest image may be confused in terms of PA and AP projections. So a "situs inversus" (heart on the other side) cannot be clearly identified.

An image gets an incorrectly-matching label:
A frequent reason for this is re-takes, where a first image chest PA has to be repeated. But the system expects the subsequent exposure "chest Lat". Therefore, when the X-ray PA is re-taken it can then be labelled as LAT.

Simple confusion: The radiographer is asked to make a hip- examination of one of the following
Standard anteroposterior hip radiograph
Frog-leg lateral view
Löwenstein view
Cross-table lateral view
False-profile view
However, the radiographer simply takes the wrong one

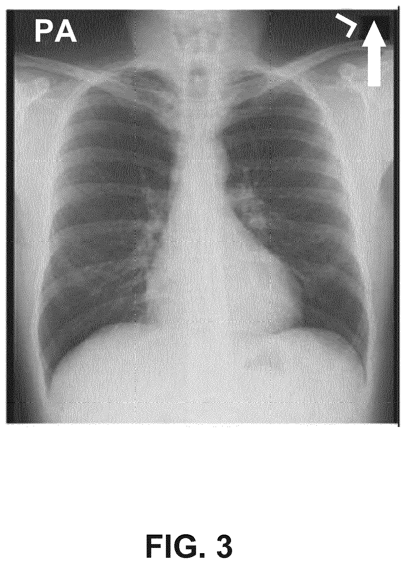

The medical image check apparatus and method of checking a medical image addresses this miss-labelling by determining a consistency between the acquired image and an associated descriptive label. Such an acquired image is shown in Fig. 3. There are two descriptive labels shown, but there can be only one. A first is "PA" shown on the left side. This text is made by a digital overlay from the DICOM projection information. So the details of this descriptive label are known and be easily provided to the apparatus. The second label is "L" shown at the right hand side. The L was a lead letter that was placed with the patient when X-rayed, and the associated imagery forms part of the X-ray image data. Thus, the descriptive label is not known as such, but can be determined from text recognition algorithms. Other labels can for example be a label for the breathing state for chest exposures: It can for example be "inhale", which is the regular case and often not explicitly shown or "exhale", which is exceptional and always requires a label.

In a detailed embodiment, the medical image check apparatus and method of checking a medical image achieves the checking of whether an image is consistent with the associated label in the following way. When an X-ray is taken, image type determination software is called that returns the known projection and anatomical feature that best matches. This process uses a local database termed the projection-database. The projection-database has been prepared in a learning phase using some many (could be thousands of) images with ground truth information.

Additionally, when a new descriptive label (Examination code like "Löwenstein view") shows up in the system via the hospital information system HIS, the system checks whether this is already in the projection-database. If this code (descriptive label) is not yet in the database a query is sent into the PACS to retrieve all available examples of this projection. If they are available, the projection-database is updated accordingly.

If the X-ray matches the descriptive label (anatomical code):
An output is displayed that the anatomy check is OK.

An output is also displayed that the laterality of the organ also matches the DICOM labels.

An output is also displayed of a list of additional views that are often combined with the one that has been done.

If the X-ray does not match the anatomical code:
An output is displayed to please check anatomy and projection!
alternative labelling is proposed
A re-take is proposed with matching positioning
Tutorial images / info are displayed
An option is display to: Accept this image as "projection OK" and update the projection-database accordingly

In another exemplary embodiment, a computer program or computer program element is provided that is characterized by being configured to execute the method steps of the method according to one of the preceding embodiments, on an appropriate system.

The computer program element might therefore be stored on a computer unit such as a smartphone, laptop, tablet, or a computer unit within an oral cleaning device such as a toothbrush, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

**1.** A medical image check apparatus (10), comprising:
- an input unit (20);
- a processing unit (30); and
- an output unit (40);
wherein, the input unit is configured to provide a radiological image to the processing unit;
wherein, the radiological image comprises image data associated with an anatomical feature of a patient;
wherein, a descriptive label is associated with the radiological image; wherein, the processing unit is configured to identify the anatomical feature in the radiological image, the identification comprising utilization of an image processing algorithm;
wherein, the processing unit is configured to determine a consistency between the identified anatomical feature in the radiological image and the descriptive label; and
wherein, the output unit is configured to output information on the basis of the determined consistency.

**2.** Apparatus according to claim 1, wherein the processing unit is configured to utilize the descriptive label to select at least one reference radiological image, and wherein the identification of the anatomical feature in the radiological image comprises a comparison between the radiological image and the at least one reference radiological image.

**3.** Apparatus according to claim 2, wherein if a determination is made that no reference radiological images are available in a local database (50), the processing unit is configured to query an external database (60) to select the at least one reference radiological image.

**4.** Apparatus according to claim 3, wherein the processing unit is configured to instruct that the local database is populated with the selected at least one reference radiological image from the external database.

**5.** Apparatus according to any of claims 1-4, wherein the radiological image comprises image data associated with the descriptive label.

**6.** Apparatus according to claim 5, wherein the image data associated with the descriptive label comprises a digital overlay.

**7.** Apparatus according to claim 5, wherein the image data associated with the descriptive label was acquired at the same time as image data associated with the anatomical feature of the patient by an image acquisition unit; and wherein the processing unit is configured to implement an image processing algorithm to identify the descriptive label.

**8.** Apparatus according to claim 7, wherein identification of the descriptive label comprises utilization of a text recognition algorithm.

**9.** Apparatus according to any of claims 1-8, wherein if the processing unit determines that the identified anatomical feature in the radiological image is consistent with the descriptive label, the processing unit is configured to control the output unit such that the output information comprises an indication that an anatomy and/or image check is ok.

**10.** Apparatus according to claim 9, wherein the output information comprises details of at least one further image view of the anatomical feature to a view associated with the descriptive label.

**11.** Apparatus according to any of claims 1-8, wherein if the processing unit determines that the identified anatomical feature in the radiological image is not consistent with the descriptive label, the processing unit is configured to control the output unit such that the output information comprises an indication that an anatomy and/or image check is not ok.

**12.** Apparatus according to claim 11, wherein the output information comprises one or more of: a request to check the anatomical feature; a request to check a view direction of an image acquisition unit; an alternative descriptive label; a request to re-take a scan with matching positioning; a display of tutorial information.

**13.** Apparatus according to claim 11, wherein the input unit is configured to receive an input from a user that the identified anatomical feature in the radiological image is consistent with the descriptive label.

**15.** A method (100) of checking a medical image, comprising:
- providing (110) from an input unit to a processing unit a radiological image, wherein the radiological image comprises image data associated with an anatomical feature of a patient, and wherein a descriptive label is associated with the radiological image;
- identifying (120) by the processing unit the anatomical feature in the radiological image, the identifying comprising utilizing an image processing algorithm;
- determining (130) by the processing unit a consistency between the identified anatomical feature in the radiological image and the descriptive label; and
- outputting (140) by an output unit output information on the basis of the determined consistency.

**16.** A computer program element for controlling an apparatus according to any of claims 1-13, which when executed by a processor is configured to carry out the method of claim 14.
